# EUROPEAN PATENT SPECIFICATION

(11) **EP 1 517 965 B1**
(45) Date of publication and mention of the grant of the patent: **28.11.2012**
(21) Application number: 03735521.1
(22) Date of filing: 03.06.2003
(51) Int. Cl.: C09C 1/36, C01G 23/047, C01G 9/02, C01G 49/02, C01G 1/02

(54) **AQUEOUS DISPERSION CONTAINING PYROGENICALLY PRODUCED METAL OXIDE PARTICLES AND PHOSPHATES**
WÄSSRIGE DISPERSION, PYROGEN HERGESTELLTE METALLOXIDPARTIKEL UND PHOSPHATE ENTHALTEND
DISPERSION AQUEUSE CONTENANT DES PHOSPHATES ET DES PARTICULES D'OXYDE METALLIQUE PRODUITES PAR PYROGENIE

(30) Priority: 03.07.2002 DE 10229761
(43) Date of publication of application: 30.03.2005
(73) Proprietor: Evonik Degussa GmbH, 45128 Essen (DE)
(72) Inventor: LORTZ, Wolfgang, 63607 Wächtersbach (DE); BATZ-SOHN, Christoph, 63454 Hanau (DE); HASENZAHL, Steffen, 63454 Hanau (DE); PERLET, Gabriele, 63538 Grosskrotzenburg (DE); WILL, Werner, 63571 Gelnhausen (DE)
(86) International application number: PCT/EP2003/005774
(87) International publication number: WO 2004/005408

(56) References cited:
- EP-A- 0 876 841
- EP-A- 1 052 225
- WO-A-90/09777
- US-A- 3 460 957

## Description

This invention relates to an aqueous dispersion containing pyrogenically produced metal oxide particles and (poly)phosphates as dispersing agent, a process for preparing this dispersion and its use in the preparation of cosmetic formulations, in particular sunscreen formulations.

Cosmetic preparations containing UV filters, such as creams or lotions, which are largely transparent on the skin and pleasant to apply, are used for the protection of the skin against excessively intensive UV radiation.

They contain as UV filters one or more organic compounds which absorb UVB radiation (290 to 320 nm) and UVA radiation (320 to 400 nm) in the wavelength range between 290 and 400 nm.

The higher-energy UVB radiation causes the typical symptoms of sunburn and the suppression of the immune defence system, while the UVA radiation, which penetrates deeper into the dermal layers, causes the premature ageing of the skin. As the combined action of the two types of radiation is reported to favour the development of light-induced skin diseases, such as skin cancer, the search for possible ways of significantly further improving the UV protection already achieved accordingly began early on.

It is known that ultrafine pigments based on metal oxides can also scatter, reflect and absorb UV radiation. Accordingly, their highly disperse formulations are an effective supplement to the organic UV filters in sunscreens.

As is generally known, ultrafine titanium dioxide is variously used for this purpose in cosmetic formulations, as it is both chemically inert and toxicologically safe and leads neither to skin irritation nor to sensitisation. It is the currently most frequently used and most important mineral light protectant. Besides titanium dioxide, ultrafine zinc oxide is increasingly being used, a distinction being made between coarse material (pigment) and fine material (micropigment). In the case of micropigments, the average primary particle size is for the most part appreciably below 200 nm, mostly within the range of 10 to 100 nm, as a rule below 50 nm.

The smallest particles obtained during the preparation of the pigments are referred to as primary particles. Primary particles may be in the form of individual crystallites or else in the form of several crystallites densely intergrown across the faces.

Particles consisting of several primary particles, where the primary particles are intergrown across the faces, are referred to as aggregates.

An agglomerate means an amalgamation of primary particles or aggregates which are held together by attractive forces such as, for example, hydrogen bridge bonds.

The coarse pigment (0.2 to 0.5 µm) absorbs or reflects the radiation widely and relatively constantly over the entire UV region and the region of visible light, whereas the fine material shows a definite increase in activity in the UV region accompanied by a simultaneous loss of activity in the long-wave UVA and in particular in the visible region. As only a little visible light is reflected, preparations based on this active ingredient are therefore largely transparent.

The metal oxides used in sunscreen formulations may exhibit an undesirable photoactivity which causes, for example, the formation of reactive species such as hydroxyl radicals. Attempts have therefore been made to suppress the formation of these species by means of inorganic and organic surface components such as, for example, Al₂O₃. SiO₂ and/or fatty acids(salts), siloxanes or phosphates.

EP-A-154150 describes the production of metal oxide particles, the surfaces of which are coated with alkyl phosphates, in an organic solvent, which after the reaction has to be removed by distillation. The particles thus obtained are used in water-repellent sunscreen formulations.

US 5,453,267 describes the preparation of sunscreen formulations containing 0.5 to 30 wt.% titanium dioxide having an average primary particle size of less than 100 nm and 0.025 to 30 wt.% phosphate anions. The titanium dioxide can be stirred into a dispersion containing the components of the formulation and the phosphate anions. Alternatively, phosphate-coated titanium dioxide particles, prepared by introducing titanium dioxide into an aqueous, phosphate-containing solution, can be used. The particles in the dispersion thus obtained are not suitable, however, for further use in a sunscreen formulation, but have to be ground several times.

In cosmetic, transparent formulations it is important that the particles be as small as possible, so that they cannot be detected on the skin just with the naked eye. At the same time, the UV protective action of a sunscreen should not be diminished and the particles should not precipitate out during storage.

To this end, the aggregated and agglomerated metal oxide particles are dispersed. By this is meant the introduction, dispersion and uniform distribution of solids in a liquid phase.

It is known that an increasingly fine division of the particles is accompanied by increased problems in dispersion, so that the dispersion process is altogether one of the most expensive steps in the preparation of cosmetic formulations. The demands of practical operation therefore render it expedient to separate this part from the preparation of the actual cosmetic formulations and to provide stable aqueous dispersions which have as high a content as possible of ultrafine metal oxide particles and preferably are of low viscosity or are at least still pumpable or flowable.

An important feature of a dispersion is the size of the dispersed particles in the dispersion. This size is referred to as the secondary particle size and describes primary particles, aggregates and agglomerates which are present in the dispersion. In contrast to a specification exclusively of the primary particle size, a specification of the secondary particle size describes the actual situation in the dispersion and in the sunscreen formulation.

The dispersion of the agglomerates and wetting of the newly created surfaces is possible with the aid of dispersing devices such as dissolvers, ball mills, Rotor-Stator machines, the degree of dispersion being dependent upon the energy introduced. The energy of these dispersing devices is insufficient for the very fine secondary particle sizes required in cosmetic and sunscreen formulations.

In EP-A-876 841 the preparation of a titanium dioxide dispersion by means of a high-energy mill is described, with an average particle size of 0.16 µm being achieved in the dispersion. The dispersion is stabilised by the addition of acetic acid. This type of stabilisation is unsuitable for cosmetic applications because, firstly, the acetic acid has a strong self-odour and, secondly, the anticipated stability in the range of ca. pH 4.5 to 7.5 relevant for cosmetic applications is low, as this range is close to the isoelectric point of titanium dioxide.

**In** WO90/09777 **titanium dioxid particles are disclosed having a mean particles size of less than 100 nm, each of the particles being coated with phosphate anions, which are suitable for use in sunscreen formulations.**

Accordingly, the object of the present invention is to prepare a highly concentrated, aqueous dispersion of ultrafine metal oxide particles, which is stable in the physiologically favourable pH range of 4.5 to 7.5 and has a low viscosity and a decreased photocatalytic activity as compared with prior art.

The object is achieved by an aqueous dispersion containing containing **aggregated,** pyrogenically produced oxide particles of titanium, zinc, iron or cerium having an average **secondary** particle size, expressed as a median value, in the dispersion of less than 200 nm, **wherein** the secondary particle sizes of the oxide particles are not distributed symmetrically in the dispersion,
**which means that the arithmetic mean of the distribution is greater than the median value and that the non-symmetrical distributions include both "oblique" monomodal and multimodal distributions**
and the dispersion contains as dispersing agent at least one (poly)phosphate corresponding to the general formula I 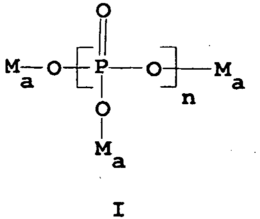 wherein
- M=: H, an alkali metal, alkaline-earth metal, ammonium ion, Zn²⁺, Al³⁺, Fe²⁺, Fe³⁺,
- a=: 1 or if M is a divalent cation, a = 1/2, if M is a trivalent cation, a = 1/3
with M being identical or different, and which has a pH value of 4.5 to 7.5.

A non-symmetrical distribution means that the arithmetic mean of the distribution is greater than the median value. Non-symmetrical distributions include both "oblique" monomodal and multimodal distributions.

The term mean value signifies the arithmetic mean of the volume-weighted particle-size distribution. The median value is the d₅₀ value of the volume-weighted particle-size distribution.

The non-symmetrical distribution of the particle sizes in the dispersion according to the invention in the presence of (poly)phosphates is surprising. In the case of the dispersion of pyrogenic, aggregated metal oxide particles, one would have expected a symmetrical normal distribution, which is identified from the fact that the ratio of mean value and median value of the distribution equals 1. Thus, for example, the dispersion of pyrogenically produced aluminium oxide in the presence of (poly)phosphates by means of high dispersive energies results in a symmetrical normal distribution.

The non-symmetrical distribution of the dispersion according to the invention means that a majority of the particles has the fineness required for cosmetic applications, while a smaller portion of coarser particles has a beneficial influence on the stability and rheology of the dispersion.

Pyrogenically produced oxide particles of titanium, zinc, iron and cerium include both oxide particles resulting from a flame hydrolysis and oxide particles resulting from a flame oxidation. During the flame hydrolysis, precursors of the metal oxides, for example, metal halides or organometallic compounds, undergo combustion in an oxyhydrogen flame, the precursor being hydrolysed. This synthesis, originally described for pyrogenic silicon dioxide, can also be used, for example, for the production of titanium dioxide. During the flame oxidation, usually metal vapour is oxidised to the metal oxide in an oxygen atmosphere. The oxidation of zinc vapour to zinc oxide may be cited as an example.

From the toxicological and dermatological aspect, the safe compounds such as cerium oxide, zinc oxide, iron oxide and in particular titanium dioxide are suitable for cosmetic formulations.

The oxide particles may also include mixed oxide particles, doped particles or coated particles of titanium, zinc, iron and cerium with one another and/or with silicon and/or aluminium. The BET surface area of the oxide particles may vary over the wide range of 5 to 200 m²/g.

The surface of the above-mentioned metal oxide particles may also be modified by means of organic compounds, so that hydrophilic or hydrophobic surfaces are obtained. Examples of metal oxide particles modified by means of organic compounds are described, for example, in DE-A-42 02 695, EP-A-1 078 957, EP-A-924 269, EP-A-722 992. The particles described in these documents can be used according to the invention, but those having hydrophobic surfaces are less suitable.

The metal oxide particles used according to the invention may, for example, be commercially available products which are obtainable under the respective trade names, also with inorganic or organic coatings such as, for example, micro titanium dioxide MT 100 AQ and MT 150 W (Tri-K-Tayca), UV-titanium M 212 (Kemira), and titanium dioxide P-25 (Degussa) and TN-90 (Nippon Aerosil). A titanium dioxide modified by organic compounds might be, for example, T 805 (Degussa).

Here, titanium dioxide P-25 (Degussa) having a BET surface area of ca. 50 m²/g and TN 90 (Nippon Aerosil) having a BET surface area of ca. 90 m²/g have proved to be particularly advantageous. The crystallographic composition of these oxides is about 80 % anatase and about 20 % rutile. They are distinguished by having high cosmetic acceptance and very good water resistance.

Preferably, the following (poly)phosphates corresponding to the general formula I are used:
alkali-metal phosphates, such as monosodium phosphate, disodium phosphate, trisodium phosphate, monopotassium phosphate, dipotassium phosphate and tripotassium phosphate; also polyphosphates, such as pentasodium triphosphate, Graham's salt, Maddrell's salt, Kurrol's salt, pyrophosphates such as tetrasodium pyrophosphate.

The (poly)phosphates may also be used in the form of mixtures. Mixtures of corresponding (poly)phosphates, which form a buffer in the pH range between 4 and 8, or more commonly between 5 and 7, are particularly preferred. Thus, for example, a 4:1 mixture of monosodium phosphate: disodium phosphate produces a buffer at about pH 6.

The dispersion may preferably contain 20 to 60 wt.% metal oxide particles. The range is particularly preferably from 30 to 50 wt.%.

The dispersion may preferably contain 0.2 to 30 wt.% (poly)phosphates corresponding to formula I. The range is particularly preferably from 0.5 to 15 wt.%.

In addition to the above-mentioned components, the dispersion may contain acids, preferably acids such as phosphoric acid, sulfuric acid, hydrochloric acid, nitric acid or carboxylic acids, for the regulation of the pH.

The dispersion may also contain known auxiliary substances and additives, such as ethanol, propanol, butanol, propylene glycol, butylene glycol, pentylene glycol, hexylene glycol, alkoxylates, glycol ethers, glycols, polyethylene glycols, polypropylene glycols, polybutylene glycols, glycerol ester ethoxylate, glycerol, polyglycerol, sorbitol, sucrose, fructose, galactose, mannose, polysorbates, starches, xanthan gum, carrageenan gum, cellulose derivatives, alginates, glycol esters, sorbitan esters, opacifiers, solubilisers, ethoxylated fatty alcohols, sodium chloride, sodium sulfate, magnesium sulfate, buffer systems, cholesterol, pantothenic acid, ascorbic acid, polyacrylic acids, carbomers.

Within the pH range of 4.5 to 7.5, the dispersion according to the invention may exhibit a zeta potential of less than -20 mV. At values of less than -20 mV, the dispersion has a particularly high stability. The zeta potential is the outwardly active potential of the particles and is a measure of the electrostatic interaction between individual particles. It is a factor in the stabilisation of suspensions and in particular of dispersions containing dispersed, ultrafine particles. At a zeta potential value of < -20 mV or > +20 mV there is a strong repulsion between the particles and the dispersions remain stable. At values within this range, the repulsion is so slight that the van de Waals forces permit the formation of agglomerates and this leads to the undesirable sedimentation of the particles.

The dispersion according to the invention may also have a viscosity of less than 2000 mPas at a shear rate of 100 s-1.

The invention also provides a process for preparing the dispersion according to the invention, which is characterised in that a stream of an initial dispersion, which contains pyrogenically produced metal oxide particles, at least one (poly)phosphate corresponding to the general formula I, water and optionally additional auxiliary substances, is divided into at least two substreams, these substreams are placed in a high-energy mill under a pressure of at least 500 bar, preferably 500 to 1500 bar, particularly preferably 2000 to 3000 bar, are released through a nozzle and impact upon one another in a gas- or liquid-filled reaction chamber and are ground.

High-energy mills are commercially available devices. For example, an Ultimaizer from Sugino or the device described in DE-A-100 37 301 are suitable for preparing the dispersion according to the invention.

The initial dispersion can be prepared, for example, by means of dissolvers, Rotor-Stator machines or ball mills. Preferably, Rotor-Stator machines are used.

The stream of dispersion can be recirculated, so that the dispersion is ground several times by means of a high-energy mill.

The dispersions according to the invention are used preferably in the preparation of cosmetic formulations, such as make-up, coloured powders, lipsticks, hair colorants, day creams and in particular sunscreen preparations and can be supplied in the conventional forms such as, for example, W/O- or O/W-dispersions (emulsions), gels, creams, lotions, sprays.

The dispersions obtained are distinguished by an extremely fine division of the dispersed solid matter, a long-term stability in storage, a low viscosity and a high photostability.

### Examples

The constituents of the dispersion according to Table 1, except for the TiO₂ particles, were placed in a receiver (batch size approx. 75 kg). The particles were then drawn in through the suction valve of an Ystral Conti-TDS 3 under shearing conditions and, when drawing-in was complete, shearing was continued at 3000 rev/min for 15 minutes (Sample 0, see Table 2). This preliminary dispersion was passed three times through the high-energy mill Sugino Ultimaizer HJP-25050 at a pressure of 2500 bar and with diamond nozzles of 0.3 mm in diameter and a sample was removed after each passage (first passage is sample 1, second passage is sample 2, and so on; see Table 2). Viscosities are shown in Table 3.

The dispersions according to the invention are stable in storage at room temperature for more than 6 months and at 50°C for more than 1 month.

**Table 1: Formulations [in wt.%]**

| | **Example** | | |
|---|---|---|---|
| | **1** | **2** | **3** |
| TiO₂⁽¹⁾ | 40 | 40 | 33 |
| sodium polyphosphate⁽²⁾ | 0.6 | - | 0.7 |
| pentasodium triphosphate | - | 0.6 | - |
| deionised water | 59.4 | 59.4 | 66.3 |
| pH value | 6.52 | 7.25 | 6.79 |
| zeta potential [mV/pH]⁽³⁾ | -29.2/5.0 | -30.5/5.2 | -27.3/5.2 |

| | | | |
|---|---|---|---|
| (1) Ex.1,2: P25(Degussa AG); Ex.3: TN90 (Nippon Aerosil); (2) Graham's salt (3) determined by means of a DT-1200 instrument from Dispersion Technology Inc., USA; | | | |

**Table 2: Particle-size distribution [in nm]¹⁾**

| | | **Example** | | |
|---|---|---|---|---|
| **Sample** | | **1** | **2** | **3** |
| 0 | median | 261 | 242 | 221 |
| | mean | 287 | 285 | 245 |
| | mean/median | 1.10 | 1.18 | 1.11 |
| 1 | median | 254 | 195 | 150 |
| | mean | 265 | 239 | 191 |
| | mean/median | 1.04 | 1.23 | 1.27 |
| 2 | median | | | 107 |
| | mean | n.d. | n.d. | 142 |
| | mean/median | | | 1.33 |
| 3 | median | 157 | 102 | 96 |
| | mean | 197 | 156 | 131 |
| | mean/median | 1.25 | 1.53 | 1.36 |

| | | | | |
|---|---|---|---|---|
| 1) determined by means of a Malvern Zetasizer 3000 HSa using the principle of dynamic light scattering. The result of the volume-weighted continuous analysis is shown. | | | | |

**Table 3: Viscosity ⁽¹⁾ of the dispersions [in mPas] relative to the shear rate [in s⁻¹]**

| | | **Example** | | |
|---|---|---|---|---|
| **Sample** | **Shear rate** | **1** | **2** | **3** |
| 0 | 1 / 100 | 10500/150 | 27500/290 | 29200/335 |
| 2 | 1 / 100 | 6900/125 | 9850/265 | 30800/425 |

| | | | | |
|---|---|---|---|---|
| (1) viscosity was determined as viscosity flow by means of a viscosimeter Physica MCR 300 and the CC27 measuring system | | | | |

## Claims

1. Aqueous dispersion containing aggregated, pyrogenically produced oxide particles of titanium, zinc, iron or cerium having an average particle size, expressed as a median value, in the dispersion of less than 200 nm, **characterised in that** the secondary particle sizes of the oxide particles are not distributed symmetrically in the dispersion,
**which means that the arithmetic mean of the distribution is greater than the median value and that the non-symmetrical distributions include both oblique monomodal and multimodal distributions**
and the dispersion contains as dispersing agent at least one (poly)phosphate corresponding to the general formula I 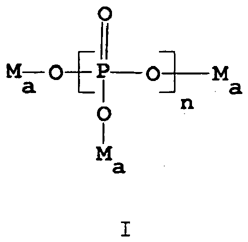 wherein
M= H, an alkali metal, alkaline-earth metal, ammonium ion, Zn²⁺, Al³⁺, Fe²⁺, Fe³⁺,
a= 1 or if M is a divalent cation, a = 1/2, if M is a trivalent cation, a = 1/3
with M being identical or different, and which has a pH value of 4.5 to 7.5.

2. Aqueous dispersion according to claim 1, **characterised in that** the metal oxide particles include the oxides of titanium, zinc, iron, cerium, mixed oxides thereof, and the mixed oxides of the above-mentioned oxides with aluminium or silicon.

3. Aqueous dispersion according to claim 1 or 2, **characterised in that** the surface of the metal oxide particles is modified by means of organic compounds.

4. Aqueous dispersion according to claims 1 to 3, **characterised in that** it contains preferably 20 to 60 wt.%, particularly preferably 30 to 50 wt.%, metal oxide particles.

5. Aqueous dispersion according to claims 1 to 4, **characterised in that** it contains preferably 0.2 to 30 wt.%, particularly preferably 0.5 to 15 wt.% of (poly)phosphates corresponding to the general formula I.

6. Aqueous dispersion according to claims 1 to 5, **characterised in that** it contains other auxiliary substances and additives.

7. Aqueous dispersion according to claims 1 to 6, **characterised in that** it has a viscosity of less than 2000 mPas at a shear rate of 100 s-1.

8. Process for preparing the dispersion according to claims 1 to 7, **characterised in that** a stream of an initial dispersion, which contains pyrogenically produced metal oxide particles, in each case at least one (poly)phosphate corresponding to the general formula I, water and optionally additional auxiliary substances, is divided into at least two substreams, these substreams are placed in a high-energy mill under a pressure of at least 500 bar, preferably 500 to 1500 bar, particularly preferably 2000 to 3000 bar, are released through a nozzle and impact upon one another in a gas- or liquid-filled reaction chamber and are ground.

9. Process according to claim 8, **characterised in that** the dispersion is ground several times by means of a high-energy mill.

10. Use of the aqueous dispersion according to claims 1 to 7 in the preparation of cosmetic formulations.

## Patentansprüche

1. Wässrige Dispersion, umfassend aggregierte, pyrogen hergestellte Oxidpartikel von Titan, Zink, Eisen oder Cer mit einer mittleren Partikelgröße, ausgedrückt als Medianwert, in der Dispersion von weniger als 200 nm, **dadurch gekennzeichnet, dass** die sekundären Partikelgrößen der Oxidpartikel in der Dispersion nicht symmetrisch verteilt sind, was bedeutet, dass der arithmetische Mittelwert der Verteilung größer als der Medianwert ist und dass die nichtsymmetrischen Verteilungen sowohl schiefe monomodale als auch multimodale Verteilungen enthalten, und die Dispersion als Dispergierungsmittel wenigstens ein (Poly)phosphat, das der allgemeinen Formel I entspricht, enthält, 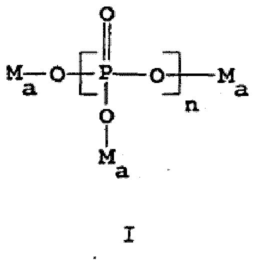 wobei
M = H, ein Alkalimetall, Erdalkalimetall, Ammoniumion, Zn²⁺, Al³⁺, Fe²⁺, Fe³⁺,
a = 1 oder, wenn M ein zweiwertiges Kation ist, a = 1/2, wenn M ein dreiwertiges Kation ist, a = 1/3,
wobei M gleich oder verschieden ist, und die einen pH-Wert von 4,5 bis 7,5 aufweist.

2. Wässrige Dispersion gemäß Anspruch 1, **dadurch gekennzeichnet, dass** die Metalloxid-Partikel die Oxide von Titan, Zink, Eisen, Cer, Mischoxide davon und die Mischoxide der vorstehend genannten Oxide mit Aluminium oder Silicium umfassen.

3. Wässrige Dispersion gemäß Anspruch 1 oder 2, **dadurch gekennzeichnet, dass** die Oberfläche der Metalloxid-Partikel mithilfe organischer Verbindungen modifiziert ist.

4. Wässrige Dispersion gemäß Ansprüchen 1 bis 3, **dadurch gekennzeichnet, dass** sie vorzugsweise 20 bis 60 Gew.-%, besonders bevorzugt 30 bis 50 Gew.-%, Metalloxid-Partikel enthält.

5. Wässrige Dispersion gemäß Ansprüchen 1 bis 4, **dadurch gekennzeichnet, dass** sie vorzugsweise 0,2 bis 30 Gew.-%, besonders bevorzugt 0,5 bis 15 Gew.-%, (Poly)phosphate gemäß der allgemeinen Formel I enthält.

6. Wässrige Dispersion gemäß Ansprüchen 1 bis 5, **dadurch gekennzeichnet, dass** sie andere Hilfsstoffe und Zusatzstoffe enthält.

7. Wässrige Dispersion gemäß Ansprüchen 1 bis 6, **dadurch gekennzeichnet, dass** sie eine Viskosität von weniger als 2000 mPas bei einer Scherrate von 100 s⁻¹ aufweist.

8. Verfahren zum Herstellen der Dispersion gemäß Ansprüchen 1 bis 7, **dadurch gekennzeichnet, dass** ein Strom einer Ausgangsdispersion, die pyrogen hergestellte Metalloxid-Partikel, in jedem Fall wenigstens ein (Poly)phosphat gemäß der allgemeinen Formel I, Wasser und gegebenenfalls weitere Hilfsstoffe enthält, in wenigstens zwei Unterströme aufgeteilt wird, diese Unterströme in eine Hochenergiemühle unter einem Druck von wenigstens 500 bar, vorzugsweise 500 bis 1500 bar, besonders bevorzugt 2000 bis 3000 bar, gegeben werden, durch eine Düse freigesetzt werden und in einer gas- oder flüssigkeitsgefüllten Reaktionskammer aufeinander prallen und gemahlen werden.

9. Verfahren gemäß Anspruch 8, **dadurch gekennzeichnet, dass** die Dispersion mehrmals mithilfe einer Hochenergiemühle gemahlen wird.

10. Verwendung der wässrigen Dispersion gemäß Ansprüchen 1 bis 7 bei der Herstellung von kosmetischen Formulierungen.

## Revendications

1. Dispersion aqueuse contenant des particules agglomérées produites par voie pyrogène d'oxyde de titane, zinc, fer ou cérium, ayant une taille moyenne de particules exprimée comme une valeur médiane dans la dispersion de moins de 200 nm, **caractérisée en ce que** la taille de particules secondaires des particules d'oxyde n'est pas distribuée symétriquement dans la dispersion, ce qui signifie que la moyenne arithmétique de la distribution est plus grande que la valeur médiane et que les distributions asymétriques incluent des distributions obliques monomodales et multimodales, et la dispersion contient comme agent dispersant au moins un (poly)phosphate correspondant à la formule générale I 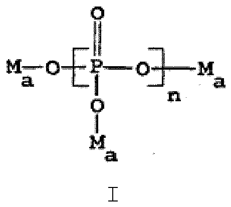 dans laquelle
M = H, un alcali, un métal, un métal alcalino-terreux, un ion ammonium, Zn²⁺, Al³⁺, Fe²⁺ ou Fe³⁺,
a = 1 ou si M est un cation divalent, a = 1/2, si M est un cation trivalent, a = 1/3,
les M étant identiques ou différents,
et qui a une valeur de pH de 4,5 à 7,5.

2. Dispersion aqueuse selon la revendication 1, **caractérisée en ce que** les particules d'oxyde métallique incluent les oxydes de titane, zinc, fer et cérium, leurs oxydes mixtes, et les oxydes mixtes des oxydes susmentionnés avec l'aluminium ou le silicium.

3. Dispersion aqueuse selon la revendication 1 ou 2, **caractérisée en ce que** la surface des particules d'oxyde métallique est modifiée au moyen de composés organiques.

4. Dispersion aqueuse selon les revendications 1 à 3, **caractérisée en ce qu'**elle contient de préférence 20 à 60 % en poids, mieux 30 à 50 % en poids de particules d'oxyde métallique.

5. Dispersion aqueuse selon les revendications 1 à 4, **caractérisée en ce qu'**elle contient de préférence 0,2 à 30 % en poids, mieux 0,5 à 15 % en poids de (poly)phosphates correspondant à la formule générale I.

6. Dispersion aqueuse selon les revendications 1 à 5, **caractérisée en ce qu'**elle contient d'autres substances auxiliaires et additifs.

7. Dispersion aqueuse selon les revendications 1 à 6, **caractérisée en ce qu'**elle a une viscosité inférieure à 2000 mPa.s à une vitesse de cisaillement de 100 s⁻¹.

8. Procédé de préparation de la dispersion selon les revendications 1 à 7, **caractérisé en ce qu'**un courant d'une dispersion initiale, qui contient des particules d'oxyde métallique produites par voie pyrogène, dans chaque cas au moins un (poly)phosphate correspondant à la formule générale I, de l'eau et éventuellement des substances auxiliaires supplémentaires, est divisé en au moins deux courants secondaires, ces courants secondaires sont placés dans un broyeur à haute énergie sous une pression d'au moins 500 bars, de préférence de 500 à 1500 bars, mieux de 2000 à 3000 bars, sont libérés par une buse, entrent en collision les uns avec les autres dans une chambre de réaction remplie de gaz ou de liquide et sont broyés.

9. Procédé selon la revendication 8, **caractérisé en ce que** la dispersion est broyée plusieurs fois au moyen d'un broyeur à haute énergie.

10. Utilisation de la dispersion aqueuse selon les revendications 1 à 7 dans la préparation de formulations cosmétiques.
